(19) **Europäisches Patentamt / European Patent Office / Office européen des brevets**

(11) **EP 1 861 713 B1**

(12) # EUROPEAN PATENT SPECIFICATION

(45) Date of publication and mention
of the grant of the patent:
**23.09.2009 Bulletin 2009/39**

(21) Application number: **06727336.7**

(22) Date of filing: **20.03.2006**

(51) Int Cl.:
*G01N 33/574* (2006.01)

(86) International application number:
**PCT/IB2006/000621**

(87) International publication number:
**WO 2006/100562 (28.09.2006 Gazette 2006/39)**

(54) **METHOD OF SCREENING PET TRACERS FOR EARLY CANCER THEREAPY MONITORING**

SCREENING VERFAHREN VON PET TRACERS ZUR FRUHEREN KREBS THERAPEUTISCHEN UBERWACHUNG

PROCEDE DE SELECTION DE TRACEURS TEP POUR CONTROLE PRECOCE DES TRAITEMENTS ANTICANCEREUX

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.03.2005 US 663818 P**
**22.09.2005 US 719726 P**
**14.03.2006 US 782261 P**

(43) Date of publication of application:
**05.12.2007 Bulletin 2007/49**

(73) Proprietor: **GE Healthcare Limited**
**Little Chalfont**
**Buckinghamshire HP7 9NA (GB)**

(72) Inventors:
• **BERGSTROM, Mats**
  **S-751 09 Uppsala (SE)**
• **RAZIFAR, Pasha**
  **S-751 24 Uppsala (SE)**
• **MONNAZZAM, Azita**
  **S-751 24 Uppsala (SE)**
• **LINDHE, Orjan**
  **S-751 09 Uppsala (SE)**
• **AWAD, Raymond**
  **S-751 09 Uppsala (SE)**
• **LANGSTROM, Bengt**
  **S-751 09 Uppsala (SE)**
• **JOSEPHSSON, Raymond**
  **S-751 83 Uppsala (SE)**
• **BLOMQVIST, Carl**
  **S-751 24 Uppsala (SE)**

(74) Representative: **Hammett, Audrey Grace**
**Campbell et al**
**GE Healthcare Limited**
**Amersham Place**
**Little Chalfont,**
**Bucks. HP7 9NA (GB)**

(56) References cited:
• **ABOAGYE ERIC O ET AL: "Preclinical development and current status of the fluorinated 2-nitroimidazole hypoxia probe N-(2-hydroxy-3,3,3-trifluoropropyl)-2- (2-nitro-1-imidazolyl) acetamide (SR 4554, CRC 94/17): A non-invasive diagnostic probe for the measurement of tumor hypoxia by magnetic resonance spectroscopy and imagi" ANTI-CANCER DRUG DESIGN, vol. 13, no. 6, September 1998 (1998-09), pages 703-730, XP009070812 ISSN: 0266-9536**
• **MONAZZAM A ET AL: "Multicellular Tumour Spheroid as a model for evaluation of [<18>F]FDG as biomarker for breast cancer treatment monitoring" CANCER CELL INTERNATIONAL 23 MAR 2006 UNITED KINGDOM, vol. 6, 23 March 2006 (2006-03-23), XP002393886 ISSN: 1475-2867 1475-2867**

## Description

## Field of the Invention

The present invention relates to a method for the screening of PET tracers in early cancer therapy monitoring. More specifically, the invention relates to a method of using multicellular tumor spheroid (MTS) model to screen PET tracers applying design of experiment (DOE) methodology. Novel semi-automated size determination method (SASDM) is provided to accurately correlate the PET tracer uptake to the size of the MTSs.

## Background of the Invention

Positron emission tomography (PET) is a multi-purpose non-invasive imaging technique with a wide range of applications both in vivo and in vitro. PET has demonstrated efficacy for monitoring therapeutic response in a wide range of cancers. In clinical oncology PET has been used for diagnosis, staging, and restaging after treatment or recurrence of different malignancies, including breast cancer. Therapy response monitoring using PET is effective because effective therapy leads to rapid physiological modification in tumours and with the right choice of PET-tracer, this modification can easily be revealed and the therapeutic response can be clarified (Cohade et al., Cancer Journal, 2002, 8(2), pp 119-134,).

With this information, physicians can quickly modify less effective therapy, thereby improving patient outcomes and reducing the cost of ineffective treatment. So far 2-[$^{18}$F]Fluor-2-deoxyglucose (FDG) has been the most common PET-tracer. PET with FDG represents a functional imaging modality that is based on metabolic characteristics of malignant tumours (van der Hiel et al., 2001, Journal of Cancer Research and Clinical Oncology, Supplement, 127(5), pp269-277). The uptake of [$^{18}$F]FDG into tissue reflects both transport and phosphorylation of glucose by viable cells. Quantitative assessment of tumour metabolism by [$^{18}$F]FDG-PET represents a novel approach in screening the response of malignant tumours to chemotherapy.

Early assessment of response would greatly benefit management of patients receiving chemotherapy by assuring continuance of effective therapy in those who respond or instituting alternative therapy in those who do not. It would also be beneficial if patients with unresponsive tumours could be identified at a much earlier stage, thereby avoiding the use of ineffective, toxic and expensive treatment. As an innovative instrument for therapy monitoring, PET provides a more timely assessment of the efficacy of specific therapies, which would then offer a significant alteration in clinical management.

However, PET with a tracer for cellular function might not necessarily allow an early assessment of treatment response. A prerequisite for this is that the treatment affects biochemical cascades leading to antitumoral actions, and that the action record by the PET tracer is in some way mechanistically coupled to these cascades. For example, inhibition of growth by alkylation of DNA might not necessarily lead to inhibition of [$^{18}$F]FDG uptake and phosphorylation. Therefore it is not advisable to start a clinical trial with an antitumoral agent expecting PET to be used for treatment monitoring, without an initial assessment that PET can indeed serve this task.

As a result, future applications of PET will likely involve other tracers in addition to FDG, to better characterize tumor biology and more effectively measure response to therapy (Gambhir, Journal of Clinical Oncology, 2005, 23(8), pp1664-1673). This potential refinement in tumor characterization will help predict clinical behavior and tailor therapy to tumor biology and thereby individualize treatment. Therefore, there is a need to develop a fast accurate screening method to select the most effective PET tracer(s) with other parameters from libraries of available PET tracers.

To monitor cancer treatment response, a suitable model is desired. In preclinical investigations, multicellular tumor spheroid (MTS) system has provided a superior and more relevant model to monolayer and is less demanding and more economical than xenograft animal model. MTSs represent a transitional level between cells growing as an in vitro monolayer and solid tumours in experimental animals or patients (Kunz-Schughart et al., Cell Biology International, 1999, 23(3), pp157-161). It has been confirmed that the cytology and the morphology of spheroids resembles that of experimental tumours in mice and natural tumours in humans before neovascularisation. In fact, MTSs represent quite realistically the three-dimensional growth and organization of solid tumors and therefore simulate the cell-cell interactions and micro environmental conditions found in the tumors (Gati et al., J. Nucl. Med. 1991, 32(12), pp2258-2265). For example, multicellular aggregates develop a central necrosis, similar to that seen in many tumors in vivo. Hence the MTS model has gained fundamental importance in therapeutically oriented investigations, as in the areas of radiotherapy, chemotherapy, photodynamic therapy, radioimunotherapy, and cell- and antibody-based immunotherapy (Gati et al., Eur. J. Cancer, 1990, 26(7), pp802-7). Aboage et al, Anti-Cancer Drug Design (1998), 13, 703-730 describes use of MTS as a pre-clinical model to demonstrate that a fluorinated 2-nitroimidazole is a probe for tumour hypoxia.

Size determination and evaluation of growth pattern are two essential aspects when studying the characteristics and behavior of the MTSs. These are important factors in studies in which e.g. the total uptake of radiotracers are evaluated and in therapeutically oriented investigations, where a drug may induce morphological changes in the tumours.

One of the most commonly used techniques for size determination of MTSs is to measure two diameters of the spheroid, using a calibrated ocular micrometer on an inverted microscope. These values are then used to determine the volume approximately. This measurement is both time consuming and imprecise, especially in the case of irregular MTSs. Moreover, MTSs tend to develop a central necrosis, which means that the volume of viable cells is quite different from the total volume of the aggregate. It is therefore necessary to have the possibility to evaluate the volume of viable cells as one entity describing the aggregate and the fraction of the total volume constituted by viable cells as another entity.

For further accuracy and precision, there is a need for an accurate method to correlate the PET-tracer uptake to the size of the MTSs. This combination gives a possibility to assess quickly, and in vitro, a good preclinical profile of existing and newly developed anti-cancer drugs.

Discussion or citation of a reference herein shall not be construed as an admission that such reference is prior art to the present invention.

## Summary of the Invention

The present invention provides a method for screening of PET tracers in early cancer therapy monitoring, comprising:

    (a) providing cultures of muticellular tumor spheroids (MTS),
    (b) treating said MTS cultures with anticancer agent(s) at a predetermined concentration for a predetermined treatment time,
    (c) determining the viable volumes of the MTSs,
    (d) incubating the MTSs with PET tracers of interest for a predetermined period of time,
    (e) measuring total PET tracer uptake for each treatment of the MTSs, and
    (f) analyzing data from PET tracer uptake per unit viable volume of the MTSs and determining suitable PET tracer(s) for each anticancer agent.

In a preferred embodiment of the present invention, the method uses design of experiment (DOE) methodology to design experiment and analyze data.

In another preferred embodiment of the present invention, the viable volumes of the MTSs are determined by semi-automated size determination method (SASDM), which comprises:

    (a) imaging a reference object,
    (b) imaging MTS,
    (c) calculating viable volume of MTS based on imaged total area of MTS, imaged area of necrosis of MTS, imaged total area of the reference object, and known total area of reference object.

Other features and advantages of the present invention will become apparent from the following detailed description. It should be understood, however, that the detailed description and the specific examples while indicating preferred embodiments of the invention are given by way of illustration only.

## Brief Description of the Figures

Fig. 1a and 1b show the significant coefficient of treatment with Paclitaxel and the predicted response measured by each tracer after treatment with Paclitaxel.

Fig. 2a and 2b show the significant coefficient of treatment with Docetaxel and the predicted response measured by each tracer after treatment with Docetaxel.

Fig. 3 a and 3b show the significant coefficient of treatment with Doxorbicin and the predicted response measured by each tracer after treatment with Doxorubicin.

Fig. 4a and 4b show the significant coefficient of treatment with Tamoxifen and the predicted response measured by each tracer after treatment with Tamoxifen.

Fig. 5a and 5b show the significant coefficient of treatment with Imatinib and the predicted response measured by each tracer after treatment with Imatinib.

Fig. 6 shows the change of viable volume of MTSs with and without 1μM of each anticancer treatment.

Fig. 7 shows the change of viable volume of MTSs with and without 10μM of each anticancer treatment.

Fig. 8 shows Competition experiment (upper left) Temperature dependence of [18F]FDG -uptake (upper right) Inhibition experiment (lower left) Insulin dependence glucose transport (lower right).

Fig. 9a and 9b show the effect of Paclitaxel in multicellular tumor spheroids of MCF-7 and BT474.

Fig. 10a and 10b show the effect of Docetaxel in multicellular tumor spheroids of MCF-7 and BT474.

Fig. 11a and 11b show the effect of Doxorubicin in multicellular tumor spheroids of MCF-7 and BT474.

Fig. 12a and 12b show the effect of Tamoxifen in multicellular tumor spheroids of MCF-7 and BT474.

Fig. 13a and 13b show the effect of Imatinib in multicellular tumor spheroids of MCF-7 and BT474.

Fig. 14 illustrates a method of using MTS model to observe effects of chemotherapy treatment on cellular growth by FDG PET.

Fig. 15 is a reference object used for absolute calibration of area. The units on the axis are number of pixels.

Fig. 16 shows images of MCF-7 cell aggregates. ROIs have been drawn separating the necrosis and living part from the background. The units on the axis are number of pixels.

Fig. 17 shows volume growth pattern for the MTS of MCF-7 cell line during 17 days of observation. The data represent the mean of 24 MTS.

Fig. 18 shows images of six different U-343 MTS. ROI have been drawn separating the necrosis and living part from the background.

Fig. 19 shows volume growth pattern for the MTS of U-343 cell line during 15 days of observation. The data represent the mean of 24 MTS.

Fig. 20 shows images of BON cell aggregates. ROI have been drawn separating the necrosis and living part from the background.

Fig. 21 shows volume growth pattern for the MTS of BON cell line during 17 days of observation. The data represent the mean of 24 MTS.

Fig. 22 shows a frozen section autoradiography of an MCF-7 MTS incubated with 18F-FDG.

Fig. 23 shows Hemotoxylin and Eosin staining of a BON MTS section. A focus of necrosis is seen in the middle part of the picture.

## Detailed Description of the Invention

The method of the present invention provides a fast and accurate method of screening PET tracers for early cancer (e.g. breast cancer) therapy monitoring. It uses a multicellular tumor spheroid (MTS) system which provides a superior and more relevant model to monolayer and is less demanding and more economical than xenograft animal model. The combination of morphological imaging and assessment of metabolic condition of tumor used in the method of the instant invention improves the quantitative knowledge about tumor and treatment response.
The method comprises of the following steps:

(a) providing cultures of muticellular tumor spheroids (MTS),
(b) treating said MTS cultures with anticancer agent(s) at a predetermined concentration for a predetermined treatment time,
(c) determining the viable volumes of the MTSs,

(d) incubating the MTSs with PET tracers of interest for a predetermined period of time,

(e) measuring total PET tracer uptake for each treatment of the MTSs, and

(f) analyzing data from PET tracer uptake per unit viable volume of the MTSs and determining suitable PET tracer for each anticancer agent.

In a preferred embodiment of the present invention, the method uses design of experiment (DOE) methodology to design experiment and analyze data.

1. Design of Experiment: In a preferred embodiment of the invention, the method starts with a design of experiment using a commercial DOE package (e.g. MODDE). Based on specific experiments, several factors can be defined. Typical factors are: anticancer agents, dose, PET tracers and treatment time. A response is also defined, e.g. tracer uptake/viable volume (of each MTS). The design experiment can then be completed by selecting the most appropriate model and design.

2. Muticellular tumor spheroid (MTS): The tumor cells are trypsinized from the stem monolayer culture. Cell suspensions are then seeded in 24-well, 1% agarose-coated culture plates, with approximately 10,000-50,000 cells per well. The cultures are kept at 37°C with 5% $CO_2$, and grown for approximately five days.

3. Anticancer treatment: The anticancer agent(s) are diluted in the growing culture medium to a predetermined concentration (from DOE). Treatment begins on the last day of MTS growth with change of the culture medium to the anticancer agent-containing medium. The medium is renewed after an hour.

In each experiment setup, four MTSs in one 24-well plate are referred to as one group. Each group includes a control (without anticancer agent) and different concentrations of the anticancer agent. Treatment time is predetermined from DOE.

4. Image analysis and size determination of MTSs: Images of MTSs are recorded by a camera (e.g. a Nikon Colorpix 4500 camera mounted on an inverted Zeiss microscope) before PET tracer incubation. Image a reference object in a similar fashion without changing any camera settings. The acquired data are analyzed by semi-automated size determination method (SASDM).

Size determination and evaluation of growth pattern are two essential aspects when studying the characteristics and behavior of the MTSs. These are important factors in studies in which e.g. the total uptake of radiotracers are evaluated and in therapeutically oriented investigations, where a drug may induce morphological changes in the tumours.

One of the most commonly used techniques for size determination of MTSs is to measure two diameters of the spheroid, using a calibrated ocular micrometer on an inverted microscope. These values are then used to determine the volume approximately. This measurement is both time consuming and imprecise, especially in the case of irregular MTSs. Additionally, MTCs tend to develop a central necrosis, which means that the volume of viable cells is quite different from the total volume of the aggregate. It is therefore necessary to have the possibility to evaluate the volume of viable cells as one entity describing the aggregate and the fraction of the total volume constituted by viable cells as another entity.

In the present invention, it provides an effective, fast and semi-automated method (SASDM) for more accurate determination of the size of MTSs and its fraction of viable cells has been developed and utilized.

A software program in Matlab (The Mathworks, Natick, Massachusetts) with user friendly interface was developed for performing the image analysis using routines from the "image processing" toolbox.

The software automatically delineates a ROI (Region of Interest) discriminating the necrosis area from the area where the cells are still alive and a ROI around the whole MCT. This is done automatically and sequentially for all images. The total area of the MTS and total area of necrosis are calculated as the total number of pixels inside the ROIs and is converted into $\mu m^2$ by using the radius of the reference object. For calculation of the volume of the MTS the following equation is used:

$$V = \frac{4}{3}\pi r^3$$

Where $r = \sqrt{area / \pi}$ is used for the calculation of the radius of the sphere. The total volume of the necrosis is calculated and subtracted from the total volume of the MTC to obtain the total volume of the living part. Additionally the fraction of total volume constituted by living cells is calculated.

All images with outlined ROIs discriminating the different parts of the MTSs are visualized and automatically saved as TIFF (Tagged Image File Format) files for further observations. All desired statistical measurements including the total volume of the whole MTSs and total volume of the necrosis part are saved in an EXCEL file with specified file name for the full group of MTSs.

5. PET tracers: To monitor the effect of each anticancer treatment, a number of established and new PET tracers can be utilized. Examples of established PET tracers are: [1-$^{11}$C]Acetate, [$^{11}$C]Choline, [$^{11}$C]Methionine, [$^{18}$F]3'-Deoxy-3'-fluorthymifine and 2-[$^{18}$F]Fluor-2-deoxyglucose.

6. PET tracer uptake: The MTS aggregates are incubated for 30 min (for $^{18}$F-labled tracers) and 50 min (for $^{11}$C-labled tracers) at 37˚C with, for example, 0.5 ml medium per well containing 3MBq tracer, and then washed 3x5 min (for $^{18}$F-labled tracers) and 3x3 min (for $^{11}$C-labled tracers) with medium (1 ml/well). Finally MTSs with washing medium are transferred to tubes and tracer uptake is evaluated in a calibrated well γ-counter. Next a volume of incubation medium is measured as reference, and an identical volume from the last wash medium is measured as background control.

The tracer-uptake in MTS is defined as:

$$\text{Tracer uptake} = (\text{Act conc. (Bq/ml) of MTS}) / (\text{Act conc. (Bq/ml) of reference}).$$

The response to each anticancer treatment is evaluated by measurement of the tracer uptake and viable volume of the MTSs.

7. Analysis of data: For each experiment, tracer uptake and tracer uptake/viable volume of MTS are normalized to the control group. All data are analyzed in a DOE program (e.g. MODDE). Analysis can use predefined factors and responses. Experimental setup can be, for example, full factorial design and the model can be computed with multiple regression.

The model can be refined by: 1. removing bad outliers (outliers for all responses); 2. removing/adding terms from/to the model that are insignificant/significant for all responses; and 3. reviewing the fit and diagnostics. Finally, a coefficient plot is generated to identify the important factors, namely, the most sensitive PET tracer(s).

## Examples

The invention is further described in the following examples which are in no way intended to limit the scope of the invention.

Example 1 - Screening PET Tracers to Monitor Early Response of Chemotherapy in Breast Cancer

In present study [1-$^{11}$C]Acetate (ACE), [$^{11}$C]Choline (CHO), [$^{11}$C]Methionine (MET), [$^{18}$F]3'-Deoxy-3'-fluor-thymifine (FLT) and 2-[$^{18}$F]Fluor-2-deoxyglucose (FDG) have been screened for monitoring the effect of five routinely used breast cancer chemotherapy agent.

Materials and Methods

1. Cell Culture

Cells of the MCF-7 human breast cancer line (European Collection of Cell Culture) were grown in MEM/EBSS supplemented with 10% FCS, 1mM sodium pyruvate, 2mM L-glutamine, 1% non-essential amino acid and 5% penicillin (Tamro). The medium was changed twice weekly and cells were maintained in exponential growth phase.

2. Multicellular Tumor Culture

The tumour cells were trypsinized from the stem monolayer culture. Cell suspensions were then seeded in 24-well, 1% agarose-coated culture plates, with approximately 50,000 cells per well. The cultures were kept at 37˚C with 5% $CO_2$, and grown for five days.

3. Anticancer Treatment

The five anticancer agents: Paclitaxel, Docetaxel, Doxorubicin, Tamoxifen and Imatinib (http://www.cancersourcemd.com/drugdb3/ )were diluted in the growing culture medium to a concentration of $1\mu M$ and $10\mu M$. Treatment begun at day six of MTS growth with change of the culture medium to the drug-containing medium. After 1 h the medium was renewed.

In each experimental set-up four MTSs in one 24-well plate were referred to as one group. The groups included control (without treatment agent) and five different treatments with same concentration.

The response to each anticancer treatment was evaluated by measurement of the tracer uptake and total volume of the MTSs after 3 and 5 days of treatment.

4. Image Analysis

Images of MTSs were recorded using a Nikon Colorpix 4500 camera mounted on an inverted Zeiss microscope before Tracer incubation. The acquired data were analyzed by SASDM.

5. PET Tracers

To monitor the effect of each anti-cancer treatment five established PET-tracers; [1-$^{11}$C]Acetate, [$^{11}$C]Choline, [$^{11}$C]Methionine, [$^{18}$F]3'-Deoxy-3'-fluorthymifine and 2-[$^{18}$F]Fluor-2-deoxyglucose were utilized.

6. PET Tracer Uptake

The aggregates were incubated for 30 min (for $^{18}$F-labled tracers) and 50 min (for $^{11}$C-labled tracers) at 37˚C with 0.5 ml medium per well containing 3MBq Tracer, and then washed 3x5 min (for $^{18}$F-labled tracers) and 3x3 min (for $^{11}$C-labled tracers) with medium (1 ml/well). Finally MTSs with 20 $\mu l$ washing medium were transferred to 5ml tubes and Tracer uptake was evaluated in a calibrated well $\gamma$-counter. Next 20 $\mu l$ incubation medium was measured as reference, and 20 $\mu l$ from the last wash medium was measured as background control.

The Tracer-uptake in MTS was defined as:

$$\text{Total uptake} = (\text{Act conc. (Bq/ml) of MTS}) / (\text{Act conc. (Bq/ml) of reference})$$

Each experiment set-up was repeated three times.

7. Analysis of Data

For each treatment, Tracer uptake and Tracer uptake/volume MTS were normalized to the control group. All data were designed and analysed in MODDE. Factors were defined as: Dose (1 and $10\mu M$), Treatment time (3 and 5 days) and Tracer (ACE, CHO, MET, FLT and FDG). Responses were defined as Uptake and Uptake/volume. Experiment set-up was designed with Full Factorial Design and the model was computed with Multiple Regression.

The Model was refined by:

1. Removing bad outliers (outliers for all responses).

2. Removing/Adding terms from/to the model that were insignificant/significant for all responses.

3. Reviewing the fit and diagnostics.

Then, to identify the important factors, coefficient plot was generated. This plot displays the coefficients for the selected response with confidence interval as error bars.

The scaling of the data makes the coefficients comparable. The size of the coefficient represents the change in the response when a factor varies from 0 to 1, in coded units, while the other factors are kept at their averages.

Results

1. Paclitaxel

A significant decrease of FLT-uptake was observed in MTSs treated with Paclitaxel, an inhibitor of microtubule remodelling.

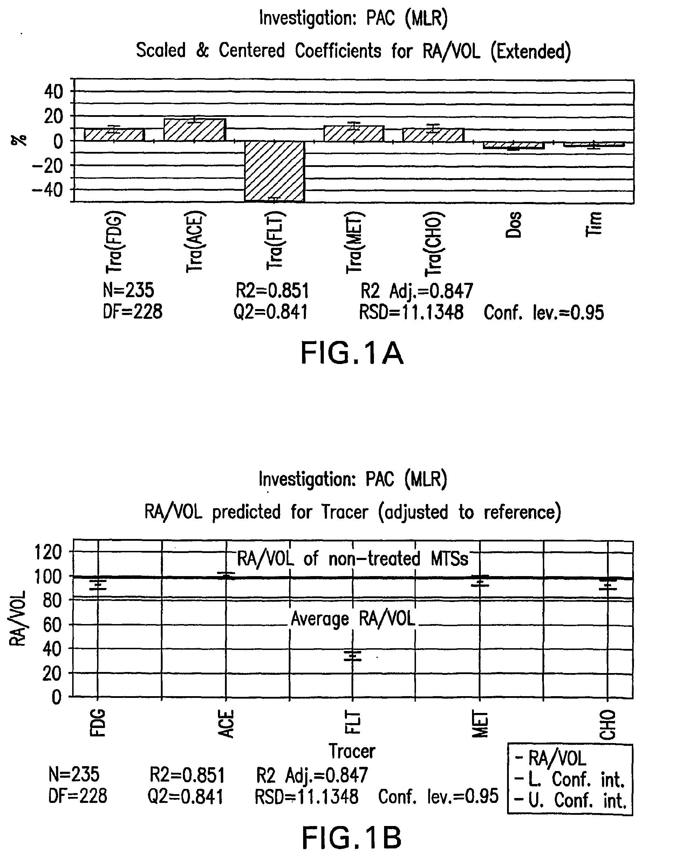

But the uptake of FDG, ACE, MET and CHO in the same group was approximately as non-treated MTSs. See Fig. 1a and 1b.

2. Docetaxel

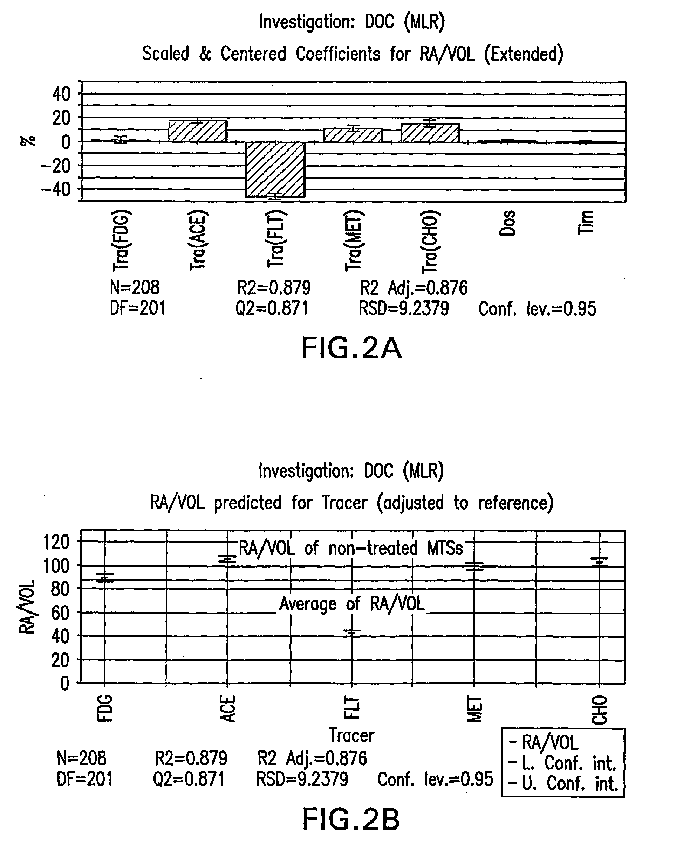

PET-tracers uptake on MTSs treated with Docetaxel, also an inhibitor of microtubule remodelling, displayed a similar pattern as Paclitaxel. See Fig. 2a and 2b.

3. Doxorubicin

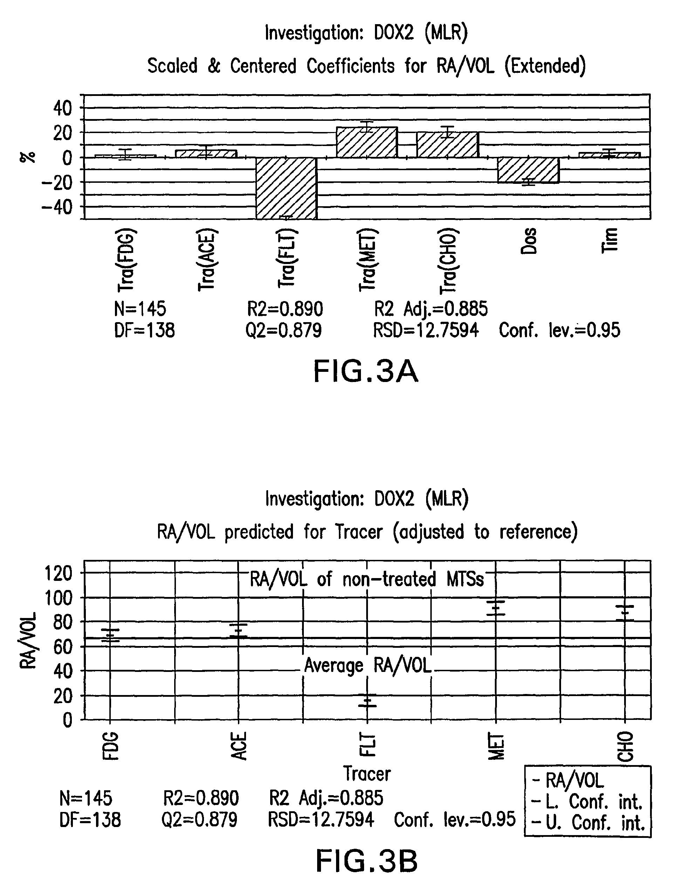

In MTSs treated with Doxorubicin, an anthracycline antibiotic, a considerable FLT-reduction and a less decrease of FDG-, ACE-, CHO- and MET-uptake was detected. See Fig. 3a and 3b.

4. Tamoxifen

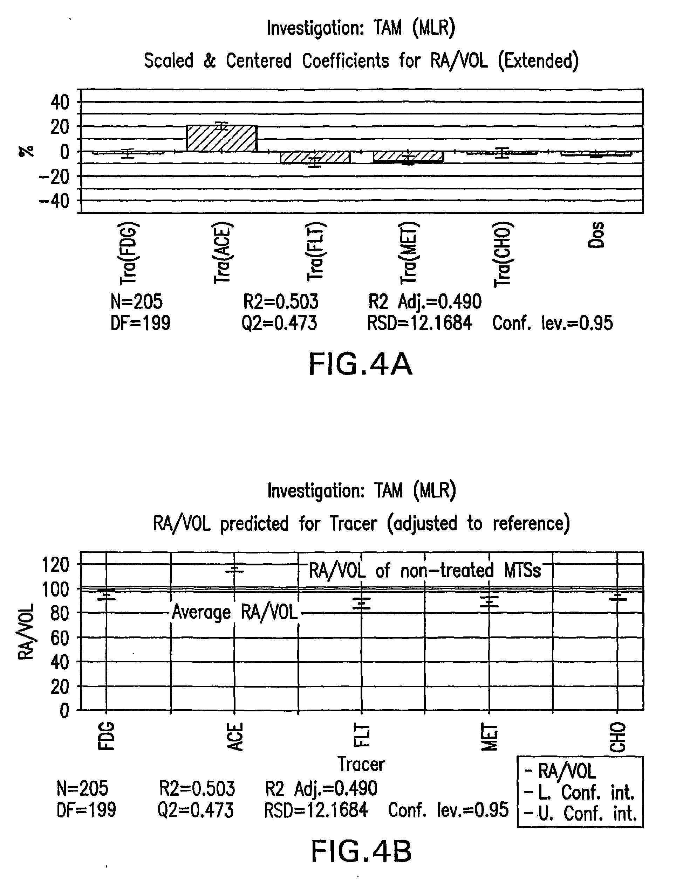

An increase of ACE-uptake was observed in Tamoxifen (an anti-oestrogen)-treated MTSs. On the other hand a decrease of FLT- and MET-uptake was observed in the same group. See Fig. 4a and 4b.

5. Imatinib

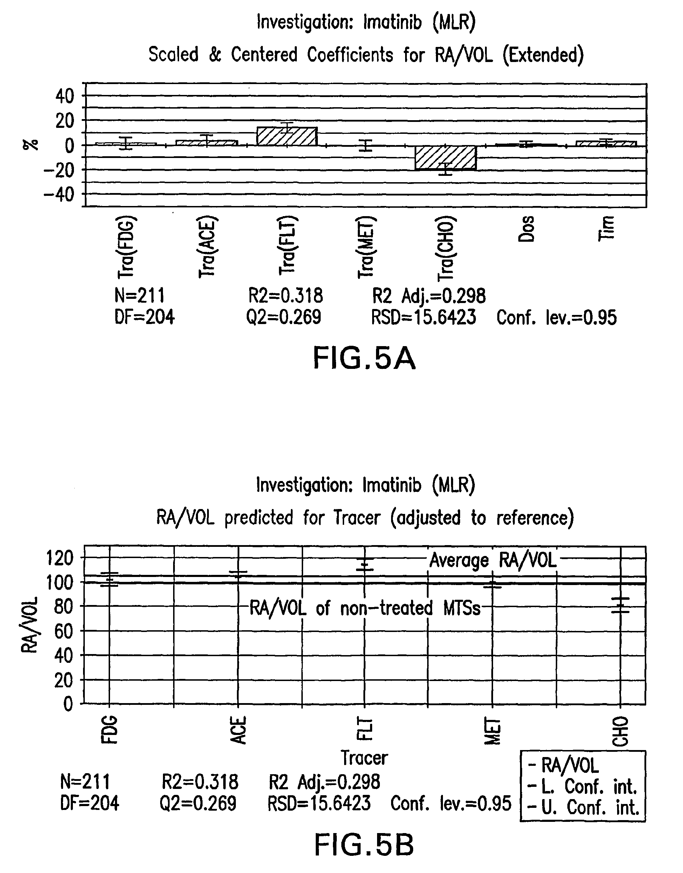

Effect of Imatinib, a protein-tyrosine kinase inhibitor, on MTSs was detected as decrease of CHO-uptake and increase of FLT-uptake. See Fig. 5a and 5b.

Discussion

The use of ACE in tumor imaging has been suggested recently in urologic malignancies. Possible biochemical paths of acetate incorporation or accumulation include (a) entering the citric acid cycle from acetyl coenzyme A (acetyl CoA) or as an intermediate metabolite, (b) esterification to form acetyl CoA as a major precursor in $\beta$-oxidation for fatty acid synthesis, (c) combining with glycine in heme synthesis, and (d) through citrate for cholesterol synthesis. Of all of these possible metabolic pathways, participation in free fatty acid (lipid) synthesis is believed to be the dominant process of incorporation in tumors. It was suggested that this mechanism of tumor uptake was different from that of myocardium, in which ACE was channeled mainly to the citric acid cycle. In our study an increase of ACE uptake was observed as a consequence of all five treatments.

In the human body, choline is needed for the synthesis of phospholipids in cell membranes, methyl metabolism, transmembrane signaling, and lipid- cholesterol transport and metabolism. Intracellular choline is rapidly metabolized to phosphorylcholine (PC); it may also undergo acetylation to form acetylcholine or oxidation to form betaine (mainly in liver and kidney). The phosphorylation is catalyzed by the enzyme choline kinase. Once phosphorylated, the polar PC molecule is trapped within the cell. Various studies have revealed an increased choline uptake as well as an up regulated activity of choline kinase and elevated levels of PC in cancer cells. In MTSs treated with Paclitaxel, Docetaxel and Doxorubicin a CHO-uptake increase was detected, on the other hand a decrease of CHO-uptake in the treatment with Imatinib, a protein-tyrosine kinase inhibitor.

MET has been used as a tumor imaging agent for several years. Its uptake reflects increased amino acid transport and, in part, protein synthesis; it also is related to cellular proliferation activity. In MTSs treated with Paclitaxel, Docetaxel and Doxorubicin a MET-uptake increase was detected, on the other hand a decrease of MET-uptake in the treatment with Tamoxifen.

FLT has been one of the promising PET agents that allow in Vivo quantification imaging of cellular proliferation. FLT is phosphorylated by thymidine kinase-1 and then trapped intracellulary by entering the salvage pathway of DNA synthesis without incorporation into the DNA molecule. FLT-uptake was decreased in relation to the treatment with Paclitaxel, Docetaxel, Doxorubicin and Tamoxifen. A FLT-uptake increase was observed in Imatinib-treatment.

Compare to the other PET-tracer, FDG, glucose transport marker, did not show high sensitivity to monitor the effect of the five chemotherapy agents.

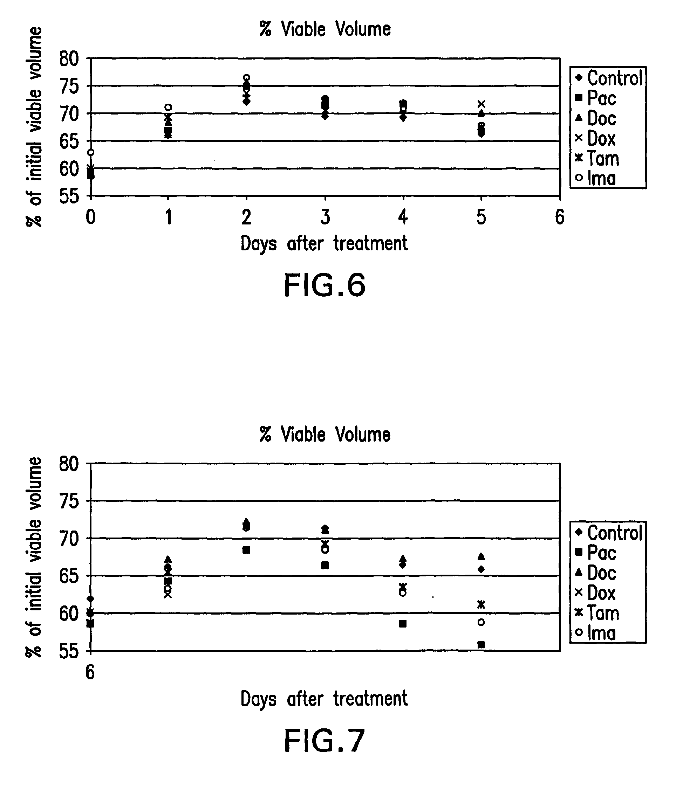

Fig. 6 and 7 show the change of viable volume of MTSs with and without $1\mu M$ and $10\mu M$ of each anticancer treatment, respectively.

Conclusion

In chemotherapy-treated tumours metabolic changes occur before anatomic changes. It means that PET can detect these changes before they can be seen with other types of imaging. But the question remains that which metabolic modifications will occur after a specific chemotherapy treatment and which PET-tracer is most sensitive to detect those particular changes.

In our study we demonstrated that for effect monitoring of different chemotherapy agents, different PET-tracers can be appropriate.

MTS model is a valuable preclinical tool to screen proper PET-tracer or combination of PET-tracers for each treatment agent(s).

Future work using other PET tracers besides FDG will undoubtedly help our understanding of tumour biology, improve our ability to measure and predict response and help tailor therapy to individual patients. More specific markers of tumour cell proliferation and gene expression may allow the application of PET not only for diagnostic imaging also but for non-invasive biological characterization of malignant tumours and early monitoring of therapeutic interventions.

This could be useful as a guide to early response to therapy as well as providing functional assessment of residual masses of disease. More specific markers of cellular proliferation e.g. [11C] thymidine, or [11C]- amino acids may provide even more accurate information.

Example 2 - MTS as a Model for Evaluation of [18F]FDG as Biomarker for Breast Cancer Treatment Monitoring

In order to explore a pre-clinical method to evaluate if [18F]FDG is valid for monitoring early response, MTS model was used to investigate the uptake of FDG in MTS with and without treatment with five routinely used chemotherapy agents in breast cancer.

Material and Methods

1. Cell lines

a. Cells of the MCF-7 human breast cancer line (European Collection of Cell Culture) were grown in MEM/EBSS supplemented with 10% FCS, 1mM sodium pyruvate, 2mM L-glutamine, 1% non-essential amino acid and 5% penicillin (Tamro). The medium was changed twice weekly and cells were maintained in exponential growth phase.
b. Cells of the BT474 human breast cancer line (American Type Culture Collection) were grown in DMEM supplemented with 10% FCS, 1mM sodium pyruvate, and 5% penicillin (Tamro). The medium was changed twice weekly and cells were maintained in exponential growth phase.

2. Multicellular tumour spheroid

The tumour cells were trypsinized from the stem monolayer culture. Cell suspensions were then seeded in 24-well, 1% agarose-coated spheroid plates, with approximately 50,000 cells per well for MCF-7 cell line and 10,000 cells per well for BT474 cell line. The spheroids were kept at 37˚C with 5% $CO_2$, and grown for five days.

3. Image analysis

Images ofMTSs were recorded and analyzed in semi-automated size determination software (SASDM). Total, necrosis and viable volume of each MTS was calculated by the program.

4. 18F-labeled 2-Fluoro-D-glucose ([18F]FDG) uptake

The MTSs were incubated for 50 min at 37˚C with 0.5 ml medium per well containing 3MBq [18F]FDG[21], and then washed 3x5 min with medium (1 ml/well). Finally MTSs with 20 $\mu l$ washing medium were transferred to 5ml tubes and [18F]FDG uptake was evaluated in a calibrated well γ-counter. A 20 $\mu l$ sample of the incubation medium was measured as reference, and 20 µl from the last wash medium was measured as background control.

5. Basic [18F]FDG experiments

To ensure the physiological validity of [18F]FDG uptake in MTS, a number of basic experiments were performed:

- *Temperature dependence of [18F]FDG uptake.* The [18F]FDG uptake experiments were performed with two different incubation temperatures: 4°C and 37°C.
- *Competition Experiment.* Glucose concentration in the growing culture medium was 5 mM. To perform the competition experiment, unlabeled extra glucose was diluted in the growing culture medium with a concentration of 0mM (for the control group), 5mM, 10mM and 20mM. Culture medium was replaced by glucose/[18F]FDG-containing culture medium. After 50 min incubation the [18F]FDG uptake was measured.
- *Inhibition Experiment.* The MTSs were pre-incubated with four different concentrations of an inhibitor of glucose transport, Cytochalasin-B; 0µM (for control group), 10µM, 25µM and 50µM followed by 50 min [18F]FDG incubation.
- *Insulin-mediated [18F]FDG uptake*. The MTSs were pre-incubated with or without (control group) 10µM insulin followed by 50 min [18F]FDG incubation.

It is noticeable that the basic experiments were performed only on MTSs of MCF-7 cell line. In each experimental set-up six MTSs in one 24-well plate were referred to as one group and the experiments were repeated three times.

6. Anticancer treatment

The anticancer agents used were:

- Paclitaxel (an inhibitor of microtubule remodelling)
- Docetaxel (also an inhibitor of microtubule remodelling)
- Doxorubicin (an anthracycline antibiotic)
- Tamoxifen (an anti-oestrogen)
- Imatinib (a protein-tyrosine kinase inhibitor).

Each drug was diluted in the growing culture medium to a concentration of 10nM, 100nM and 1µM. Treatment began at day five of MTS growth with change of the culture medium to the drug-containing medium. After 1 h the medium was renewed, and the MTSs remained in drug containing medium for 2-3 days.

For the MCF-7 cell line, each group included 6 replicates and the experiments were repeated twice.

For the BT474 cell line, each group included 4 replicates and the experiments were repeated three times.

The response to each anticancer treatment was evaluated by measurement of the [18F]FDG uptake and viable volume of the MTSs after 2 and 3 days of treatment.

7. Immunohistochemistry

$\gamma$-H2AX

8. Analysis of data

[18F]FDG uptake per viable volume of each MTS was normalized to the control group. All data were analysed by ANOVA and Dunnett's Multiple Comparison Test in Graph Pad Prism.

Results

The tumour cells formed multicellular spheroids about 1 day after seeding. Within the next day the characteristic rounded shape with a rim of viable cells and a central necrosis was observed.

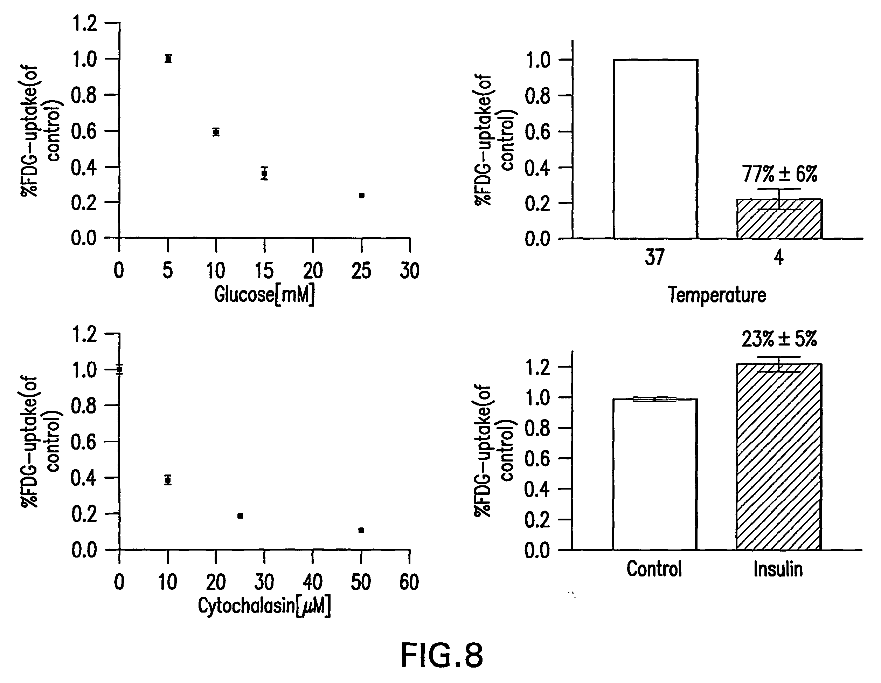

When an increasing concentration of glucose was added to the incubation medium, [18F]FDG uptake in MTSs gradually decreased (Fig 8). The concentration inducing a 50% reduction compared to control was 10 mM. A reduced temperature during the [18F]FDG incubation reduced the [18F]FDG uptake by 77% (SE=6%)(Fig. 8). These two observations indicate a saturable and temperature-dependent process for the [18F]FDG uptake and hence exclude dominance by passive diffusion.

When the glucose-transport inhibitor Cytochalasin B was added to the incubation medium, [18F]FDG uptake significantly decreased; e.g., a 50% inhibition occurred at 12 µM (Fig 8). Addition of insulin to the incubation medium

increased the $[^{18}F]$FDG uptake by 23% (SE=5%) (Fig 8). These two observations suggest glucose transport is the dominant mechanism for $[^{18}F]$FDG uptake in the MTSs.

1. Paclitaxel and Docetaxel

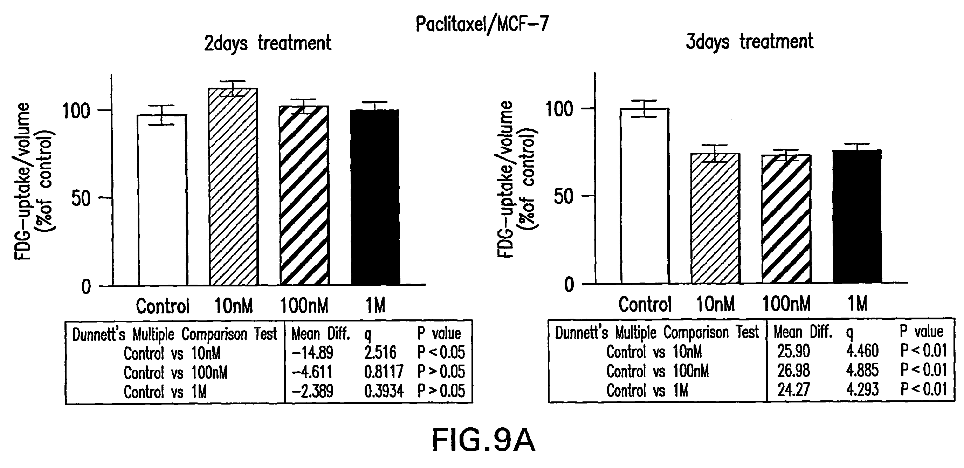

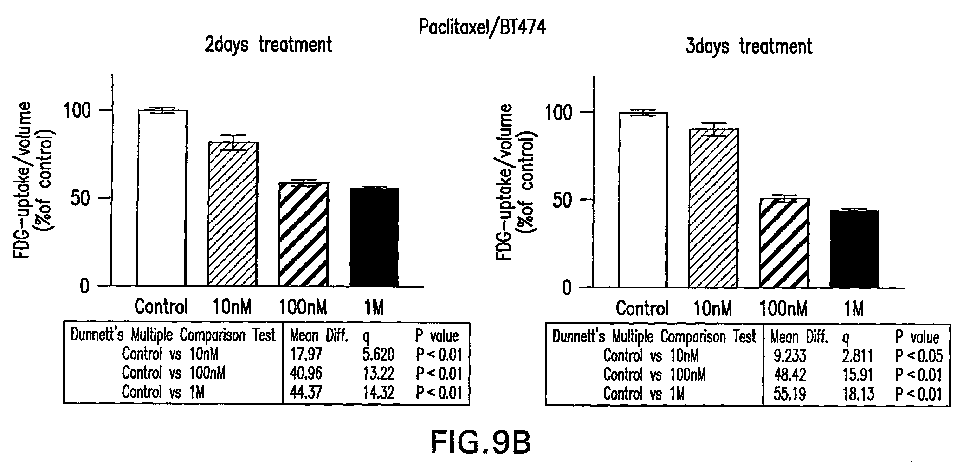

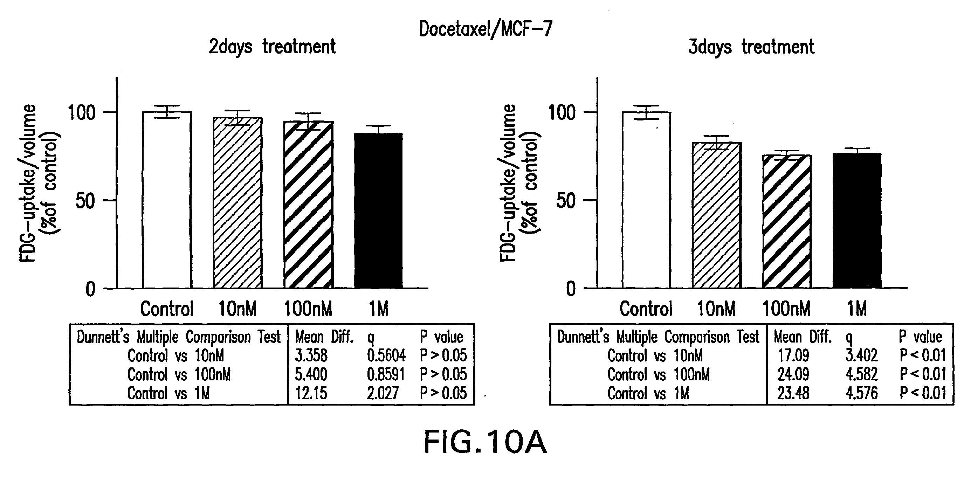

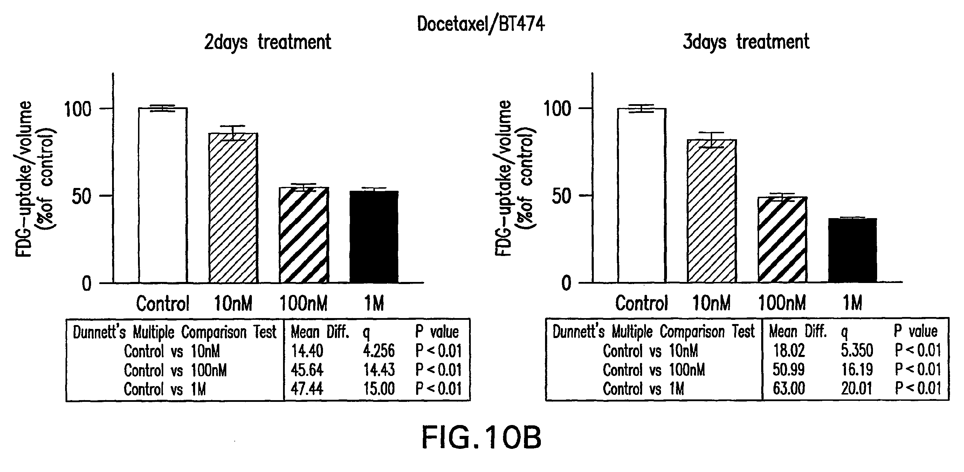

Taxanes significantly reduced the uptake of $[^{18}F]$FDG. The decrease in uptake per viable volume was more evident in BT474 (up to 55% decrease) than MCF-7 (up to 25% reduction) (Fig 9a, 9b, 10a and 10b).

2. Doxorubicin

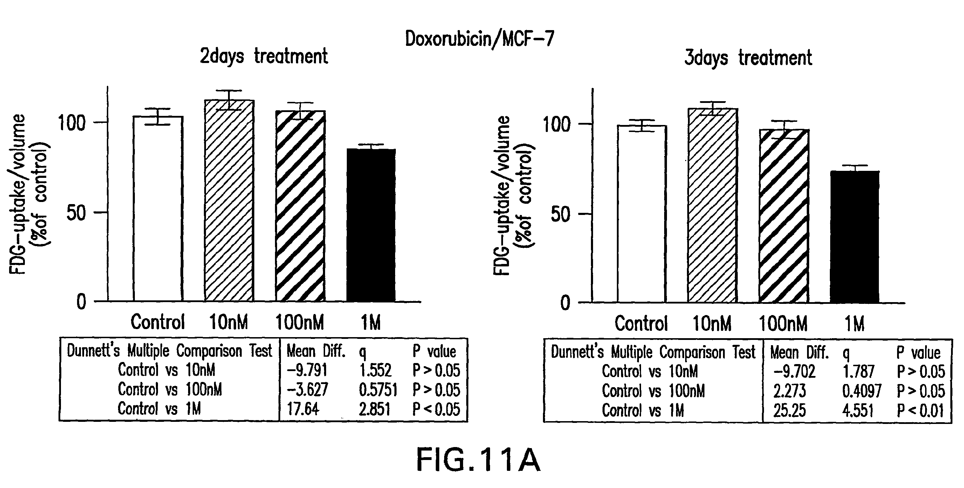

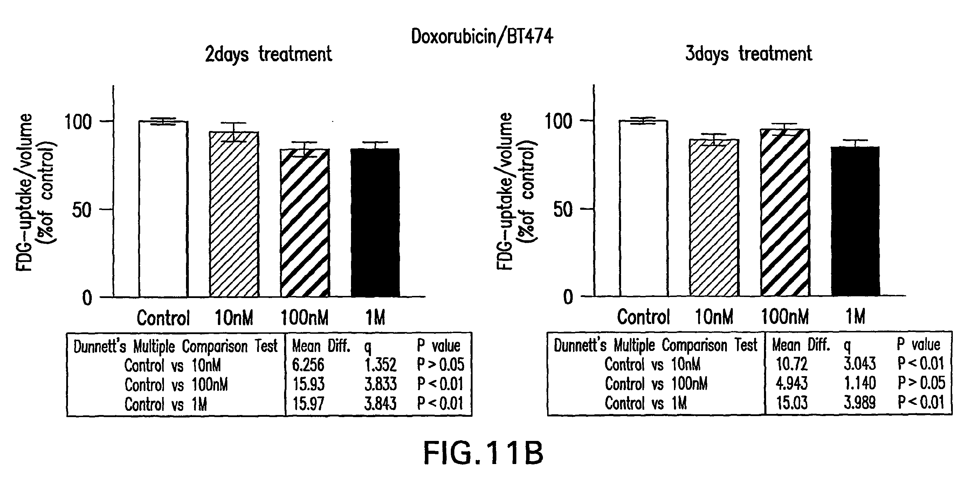

Doxorubicin, which damages DNA by intercalation, reduced $[^{18}F]$FDG uptake per viable volume at the highest dose, more noticeable in MCF-7 (25%) than in BT474 MTSs (Fig. 11a and 11b).

3. Tamoxifen

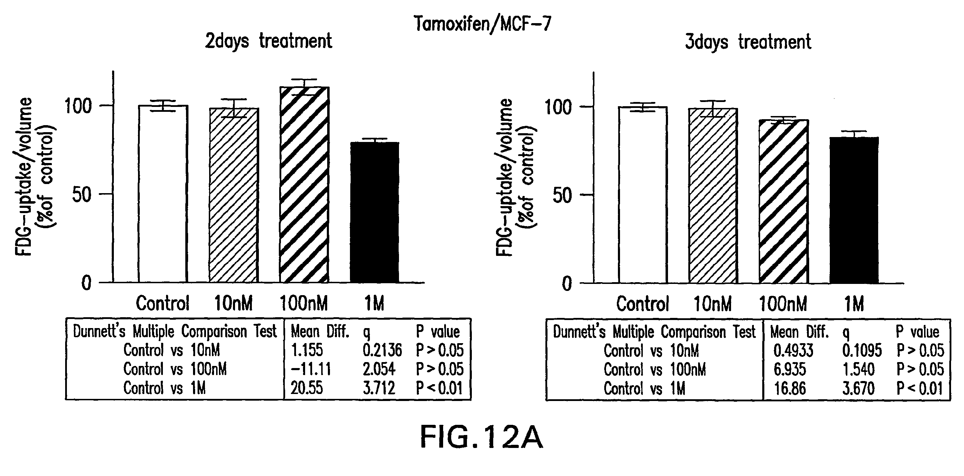

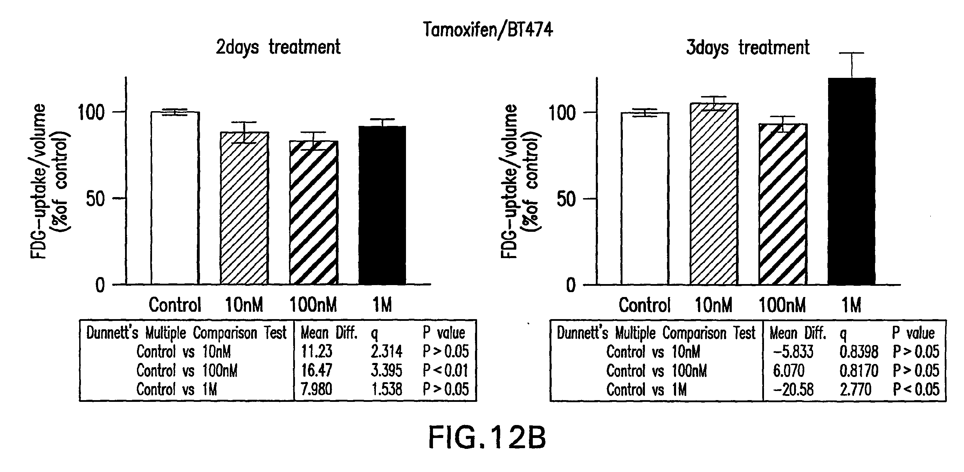

Tamoxifen, an anti-oestrogen compound, reduced $[^{18}F]$FDG uptake per viable volume in MCF-7 at the highest dose of 1 $\mu$M (Fig. 12a and 12b).

4. Imatinib

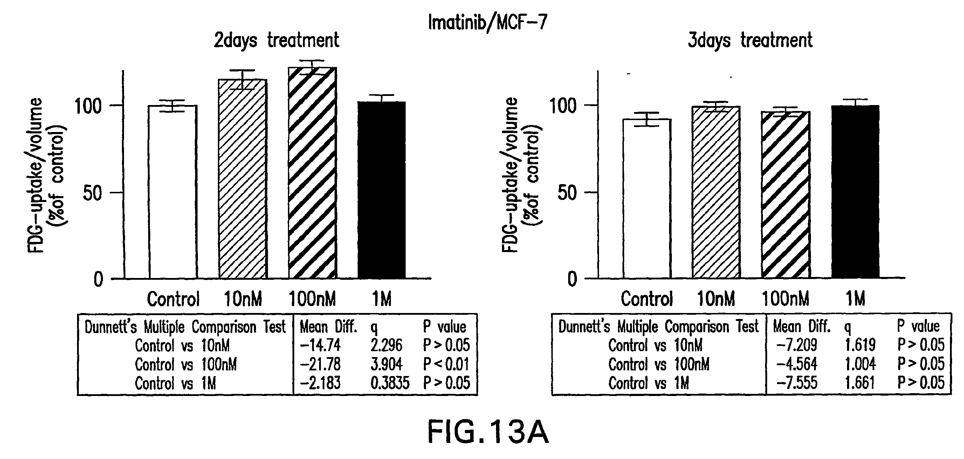

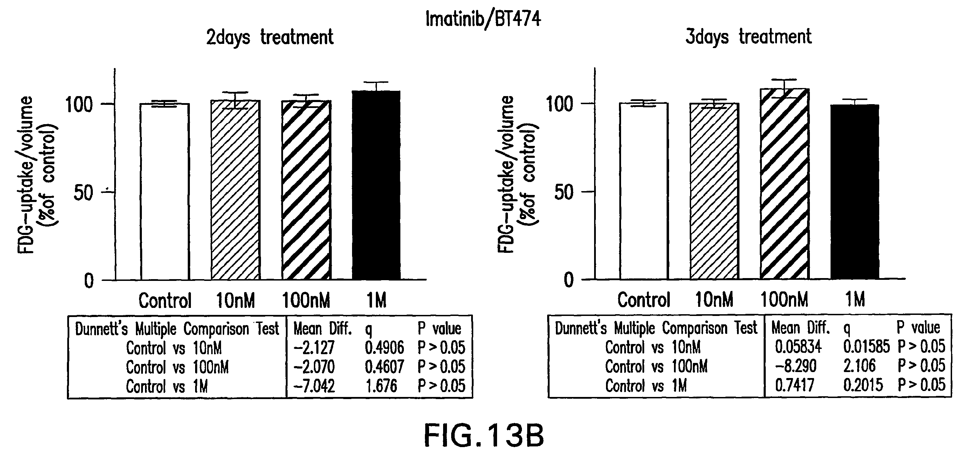

Imatinib, a tyrosine kinase inhibitor, slightly increased $[^{18}F]$FDG uptake in MCF-7 at the intermediate dose of 100 nM and 2 days follow up, otherwise no effect was observed (Fig. 13a and 13b).

Discussion

In order to explore if a range of conventional anti-cancer drugs would impact on the cellular uptake of FDG, we utilized multicellular tumour spheroids, treated with the drugs and after a few days of treatment evaluated the uptake of FDG. Two different breast cancer cell lines were selected with different characteristics. BT474 cells have up-regulated mRNA and HER2/neu tyrosine kinase-linked receptor protein in comparison to MCF7, while the expression of the estrogen receptor alpha is know to be up-regulated in MCF7 cells. These two cell-lines are assumed to be representative for a variety of breast cancers.

Although growing cells as MTS is more laborious than monolayer, MTS cells more closely mimic the real biological environment by providing cell-to-cell adhesion and signalling. This gives a more relevant picture of the drug effects by including limitations in penetration, distribution and feedback mechanisms in cell signalling.

Here we established a new methodology for potential drug screening by combining measurement of the morphologic drug effect and evaluation of PET tracer uptake. The method could serve dual purposes: to evaluate effects of new drugs and to identify the optimal PET biomarker for early evaluation of drug effect.

Initially we investigated the nature of $[^{18}F]$FDG uptake in MTS. We verified a reduced metabolism after either challenging with low temperature, or competition by unlabeled glucose or inhibition with Cytochalasin-B. We also observed a transport increase with insulin addition. These sets of tests confirmed that in MCF-7 MTSs, $[^{18}F]$FDG accumulation is a biomarker which is related to transport via the specific glucose transporters GLUT, followed by phosphorylation by hexokinase.

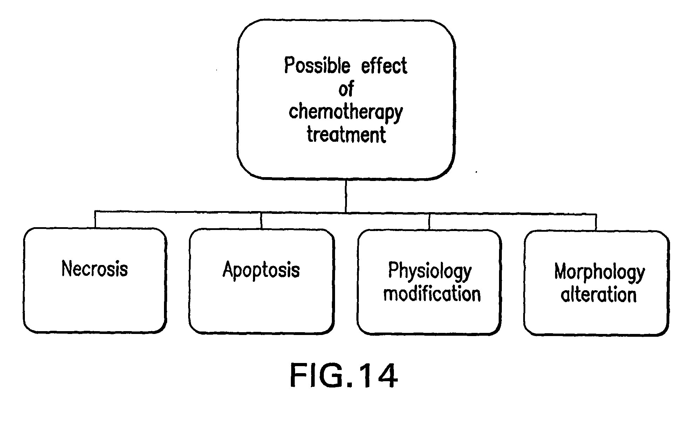

An important aspect in the evaluation of chemotherapy by PET-FDG is whether $[^{18}F]$FDG can be used as a biomarker to indicate treatment response. The accepted criterion for treatment response is reduction in tumour size or verified lack of further growth. This can in turn relate to a variety of functional aspects, such as induction of apoptosis, direct cell kill, effects on vascularity, reduced proliferation, remodelling of proportions of tumour and other cells etc. These aspects do not necessarily relate to effects on FDG uptake. For example, transient increase in uptake of FDG has been postulated to be an indication of a positive effect causing a temporary inflammatory response, metabolic flare. A retardation of cellular proliferation can be associated with a lack of effect on FDG uptake. Hence it is of utmost importance to clarify the relation between antitumoral effects and effects on FDG uptake. For this, we believe that MTS are the best in vitro cellular model, allowing an easy evaluation of FDG uptake plus a means to observe effects on cellular growth. The method is ideally associated with evaluations of apoptosis induction, proliferation and other cellular biomarkers (Fig. 14).

Paclitaxel and Docetaxel has a known activity against a broad range of tumour types, including breast, ovarian, lung, head and neck cancer. These potent antineoplastic drug binds to the N-terminal region of b-tubulin and promotes the formation of highly stable microtubules that resist depolymerization, thus preventing normal cell division and arresting the cell cycle at the G2-M phase. Combining $[^{18}F]$FDG-PET and image analysis, we monitored the effect of Paclitaxel for 3 days on two different human breast cancer line, in a concentration of 10nM, 100nM and 1$\mu$M, added after 5 days of growth as MTS. In BT474, we observed reduced glucose transport per viable volume already at the lowest dose and the shorter treatment time. The effect was more moderate in MCF-7. These results indicate disturbance in cells physiology as a consequence of the treatment with taxanes that can be recorded with [$^{18}$F]FDG-PET.

Doxorubicin is an anthracycline antibiotic produced by the fungus streptomyces peucetius. It damages DNA by intercalation of the anthracycline portion, metal ion chelation, or by generation of free radicals. Doxorubicin has also been shown to inhibit DNA topoisomerase II which is critical to DNA function. Cytotoxic activity is not specific to cell cycle phase. Doxorubicin is also known to be activated by mitochondrial electron transport system and causes mitochondrial energy failure. Thus, the effect might be not only by DNA damage, but also by energy metabolism damage. In our experiments Doxorubicin decreased glucose transport at the highest dose. The data suggest that [$^{18}$F]FDG would record an antitumoral effect of Doxorubicin in the early phase of treatment at relevant doses. The effect of Doxorubicin was more intense on MCF-7 than on BT474.

Tamoxifen is a synthetic non-steroidal anti-oestrogen. It is thought to competitively block oestrogen receptors. Other biochemical effects of Tamoxifen include interaction with protein kinase C and stimulation of human NK cells. As expected, the effect of Tamoxifen can only be observed in MCF-7. In our experiments the effect of Tamoxifen can only be related to a glucose-transport alteration.

Imatinib mesylate is a protein-tyrosine kinase inhibitor that inhibits the Bcr-Abl tyrosine kinase, the constitutive abnormal tyrosine kinase created by the Philadelphia chromosome abnormality in chronic myeloid leukemia (CML). It inhibits proliferation and induces apoptosis in Bcr-Abl positive cell lines as well as fresh leukemic cells from Philadelphia chromosome positive chronic myeloid leukemia.

Imatinib is not entirely selective; it also inhibits the receptor tyrosine kinases for platelet-derived growth factor (PDGF), stem cell factor (SCF), c-Kit and thereby inhibits PDGF- and SCF-mediated cellular events. In our experiments no effect of Imatinib was observed.

Conclusions

To conclude, the combination of PET radiotracers, image analysis of growth pattern and necrosis induction plus histochemical analyses of proliferation and apoptosis in MTSs provides a good model to evaluate the relationship between tumour volume and the uptake of metabolic tracer before and after chemotherapy. This feature could be used for screening and selecting PET biomarkers for early assessment of treatment response.

In addition, this new method gives a possibility to assess quickly, and in vitro, a good preclinical profile of existing and newly developed anti-cancer drugs. Used clinically on biopsies, this method could potentially be used on an individual level, first to select a treatment for a particular patient, and then to select the PET tracer for monitoring in a short follow up time the optimal drug and dose regimen.

Example 3 - SASDM for Studying MTSs

In present study an effective, fast and semi-automated method (SASDM) has been developed to determinate the size of MTSs. The method was applied and tested in MTSs of three different cell-lines. Frozen section autoradiography and H&E staining was used for further confirmation.

Materials and Methods

1. Cell culture

Three standard cell lines were used for investigation of the performance of this method;

- MCF-7 a human breast cancer cell line (European Collection of Cell Culture).
- U-343 a human glioma cell line.
- BON a human neuroendocrine tumour cell line (a kind gift from Dr. C.M Townsend, University of Texas, Galvestone, USA) derived from a lymph node metastasis of a pancreatic carcinoid.

The MCF-7 cells were grown in MEM-Eagle medium supplemented with 10% FCS, 1mM sodium pyruvate, 2mM L-giutamine, 1% non-essential amino acids and 5% Penicillin (Tamro). U-343 cells were cultured in Ham's F-10 medium supplemented with 10% FCS, 1mM sodium pyruvate and 2mM L-glutamine (Tamro). BON cells were grown in Ham F-12K medium (NordCell, Sweden) mixed with DMEM medium supplemented with 10% FCS, 1mM sodium pyruvate and 2mM L-glutamine (Tamro).

The medium was changed twice a week and the cells were maintained in exponential growth phase.

2. Multi-cellular tumour culture

The tumour cells were trypsinized from the stem monolayer culture, then cell suspensions were seeded in 24-well 1% agarose coated culture plates, with approximately 50.000 cells per well for U-343 and MCF-7 and 15.000 for BON. The cultures were kept at 37˚C with 5% $CO_2$ and grown for a period that was depended on duplication rate for each cell line.

MTS medium for MCF-7 was DMEM supplemented with 10% FCS, 1mM sodium pyruvate, 2mM L-glutamine, 1% non-essential amino acids, 5% Penicillin (Tamro), 0.01 mg/ml Insulin and 1nM β-Estradiol (Sigma Aldrich).

3. Image Analysis

The aggregates were photographed daily using a Nikon Colorpix 4500 (www.nikon.com) digital camera mounted on a Zeiss Axiovert 135 Microscope (www.zeiss.com). The digital camera was set to utilise up to 4.0 million effective pixels, with an image resolution of 640x480, auto-focus, auto-shooting modes, with applied "Hi" image quality.

A 5x magnification objective, 5x/0,12; 44 01 20 on Zeiss Axiovert 135 Microscope was used with adjustment of the strength of background light depending on the color of the medium that was used for growing the desired MTS.

Images were saved as sequences of JPEG files in a 128 MB, Scan Disc Compact Flash card and transferred into the hard disc of a personal computer with installed windows 2000 for further image and statistical analysis.

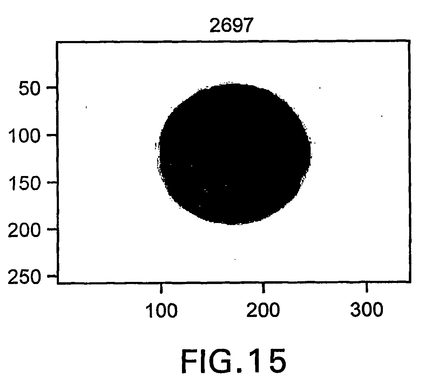

A software program in Matlab (The Mathworks, Natick, Massachusetts) with user friendly interface was developed for performing the image analysis using routines from the "image processing" toolbox. For absolute calibration of the volume a spherical object with a specified diameter of 0.79375 $\mu m$ as shown in Fig. 15, was used as reference obj ect.

After feeding some input parameters such as name of starting image, number of images to analyze, project name etc, the software automatically delineates a ROI (Region of Interest) discriminating the necrosis area from the area where the cells are still alive and a ROI around the whole MTS. This is done automatically and sequentially for all images. The total area of the MTS and total area of necrosis are calculated as the total number of pixels inside the ROIs and is converted into $\mu m^2$ by using the radius of the reference object. For calculation of the volume of the MTS the following equation is used:

$$V = \frac{4}{3}\pi r^3$$

where $r = \sqrt{area / \pi}$ is used for the calculation of the radius of the sphere. The total volume of the necrosis is calculated and subtracted from the total volume of the MTC to obtain the total volume of the living part. Additionally the fraction of total volume constituted by living cells is calculated.

All images with outlined ROIs discriminating the different parts of the MTSs are visualized and automatically saved as TIFF files for further observations. All desired statistical measurements including the total volume of the whole MTSs and total volume of the necrosis part are saved in an excel file with specified file name for the full group of MTSs.

The growth curves for the three selected cell lines were generated to illustrate the use of the program. During the first few days of culture after seeding, the aggregates settle, which means they do not follow an exponential growth pattern. The growth curves after this period of 5-9 days were plotted and data fitted to a monoexponential to indicate the volume doubling time.

4. Frozen Section Autoradiography and Staining

At a selected time point some of the MTSs were incubated with $^{18}$F-labeled Fluoro-Deoxy-Glucose(FDG) to confirm the extent of the necrosis part. After 40 minutes incubation with 100 MBq/ml FDG and washing for 3*5 minutes, the MTSs were frozen quickly at -20˚C and sectioned for autoradiography.

The slices with a thickness of 25$\mu m$ were exposed on a phosphor-imaging plate for a period of 20 hours. Scanning and imaging were performed using software Image Quant (Molecular Dynamics, Sunnyvale, CA).

For further verification Hematoxylin & Eosin staining of the slices was utilized. This part was a complementary part to clarify some histological features in the MTSs.

Results

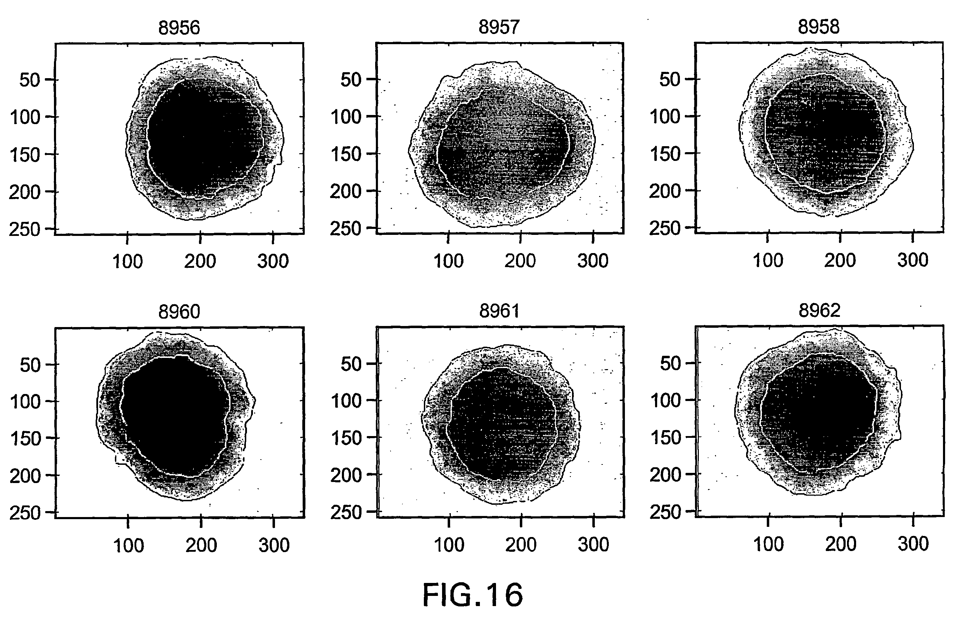

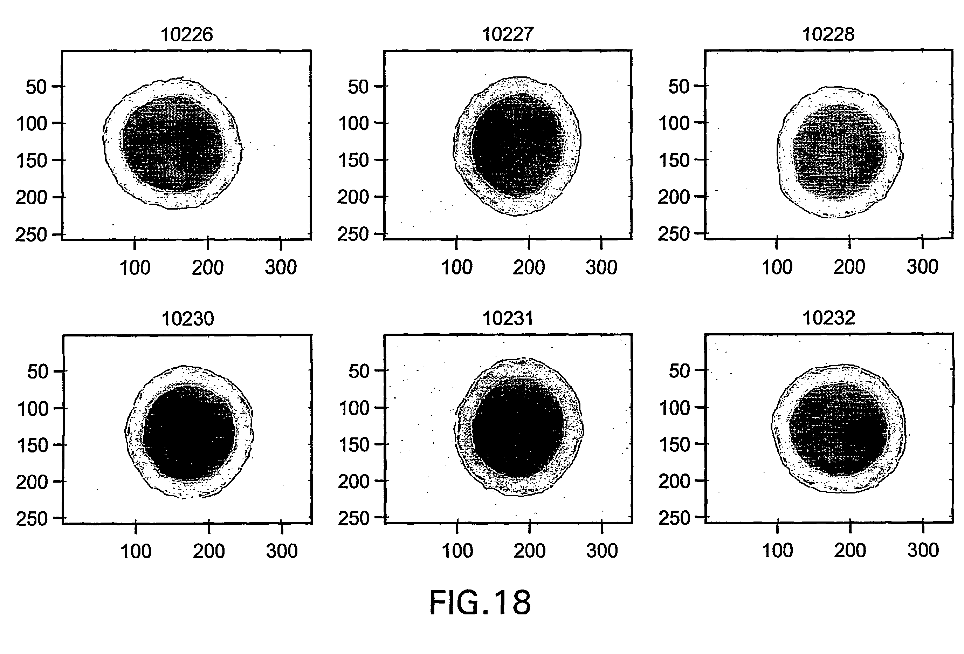

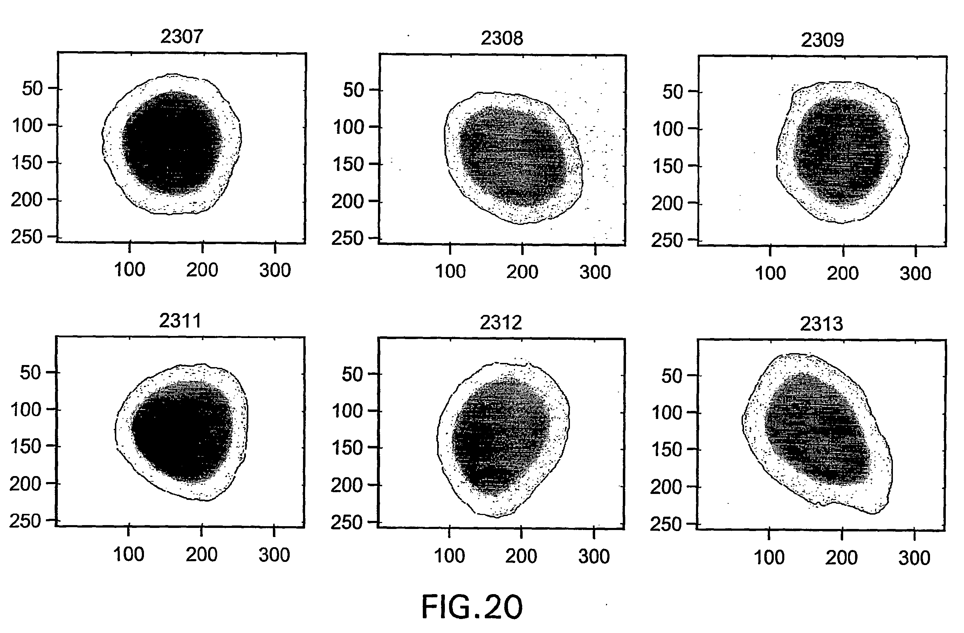

Multicellular aggregates from all cell lines showed directly after seeding a uniform feature without necrosis, which after a period of a few days changes to the typical feature shown in Fig. 16, 18 and 20, with a rim of viable cells surrounding a central necrosis. The shapes were slightly different and the sizes of individual aggregates also different. The automatic outlining of the necrosis and the total periphery behaved properly in all cases.

The computation time for a set of 25 images was 17 seconds.

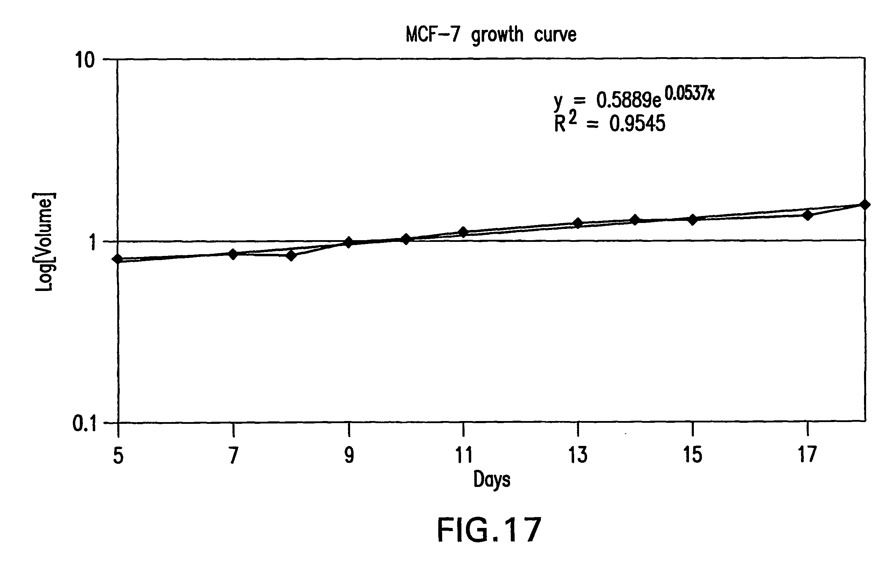

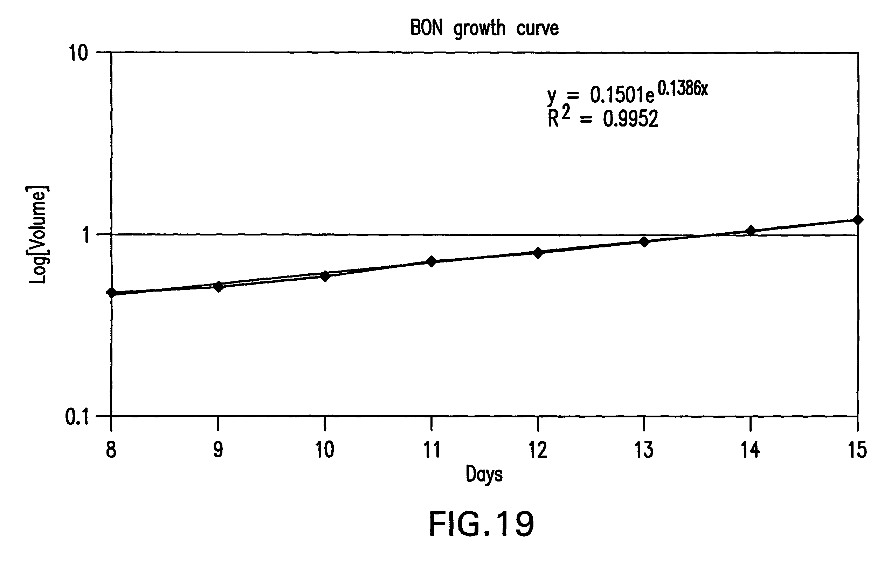

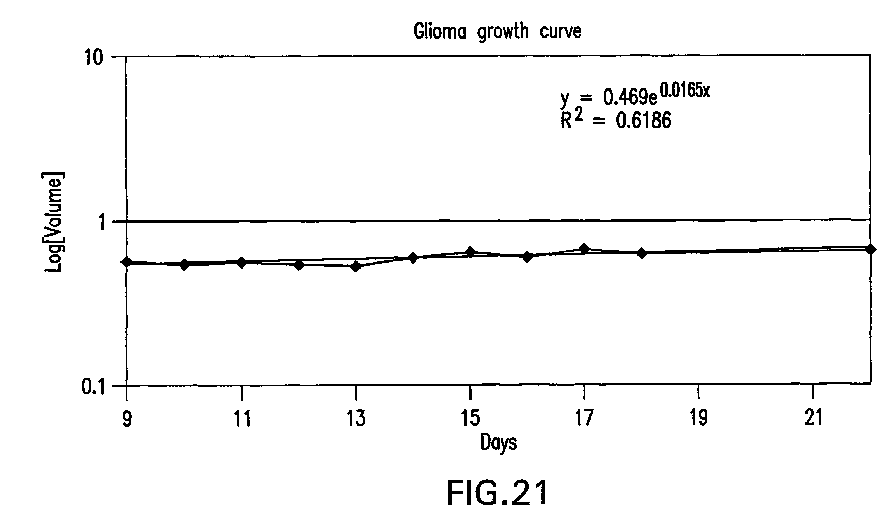

The MTS volume doubling time was calculated to be13days for MCF-7 (Fig. 17), 57 days for U-343 (Fig. 19) and 5 days BON (Fig. 21).

For each cell line, the variability within the group was below 15% (COV) but considerably higher between groups. This is another confirmation of how essential is to determinate the size of MTS for each experiment set-up.

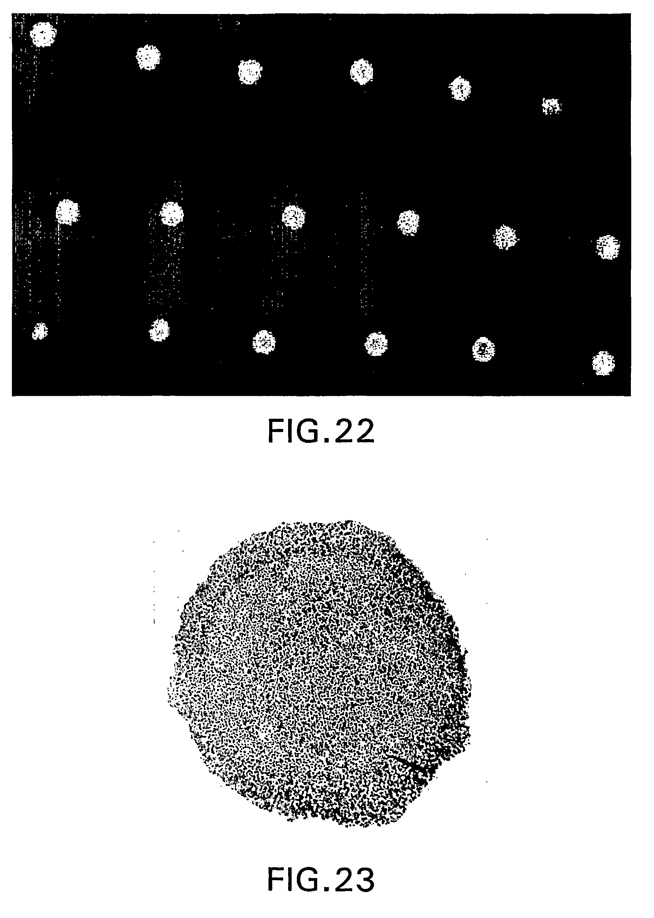

The result of autoradiography as well as the result of H&E staining confirmed the necrosis part in the middle of the MTSs (Fig. 22 and 23).

Discussion

The traditional method to use cultured tumor cells for biology evaluations, including assessment of radiotracer uptake and effects of treatment, utilizes monolayer culture. This method is easy to apply, allows a good and reproducible source of cells and allows easy calculation of cell number by counting with more or less sophisticated methods. However, there are some clear drawbacks to the method, especially with respect to evaluation of radiotracer uptake and monitoring of treatment response. The primary issue is that the growth is severely up-regulated in monolayer as compared to in vivo growth due to lack of adequate contact inhibition. This accelerated growth and lack of direct communication with neighboring cells also questions the physiological relevance, with the expectation that cellular function is aberrant. Furthermore, monolayer with its rapid growth, very quickly change from a sparse cell culture to a culture with overlay growth, and thereby prohibit long term follow up of normal growth pattern compared to growth with pharmacological modulation. To overcome this, it is customary to only study short term effects, or to expose the cells to trypsinization and reseeding, a process which can induce further changes to cellular function.

To overcome these and to have additional advantages for radiotracer uptake studies, we have since long been working with multicellular aggregates, grown on agar as a method to have easy access to cells. This method has the additional advantage that it allows an easy washing of the aggregates followed by a measurement of radiotracer uptake, and thereafter the aggregates can return to culture for further later evaluations if the radiotracer is a PET tracer with short half-life.

A prerequisite for a proper evaluation of cell physiology with PET tracers is that the uptake value is related to the amount of living cells, especially if it is desired to have a proper measure to be translated to an in vivo situation, respectively in evaluations of treatment effects where changes in the relative proportion of viable cells can be expected. We have therefore developed a routine including quantization of PET tracer uptake relying on an accurate measurement of viable cell volume.

The introduced method is very effective, user-friendly, time-saving and more accurate than the traditional method. It is known that a tumor cell-line can behave different under different conditions. One of the advantages of this method is that a growth-curve can simply be generated for each cell-line in each laboratory and for each experimental set-up.

Combining (SASDM) with biological evaluation of the MTS provides a good model to evaluate the effect of various cancer treatments e.g. chemotherapy and radiotherapy.

Conclusion

To conclude, the combination of PET radiotracers and image analysis in MTSs provides a good model to evaluate the relationship between viable volume of tumour and the uptake of metabolic tracer before and after chemotherapy. This feature could be used for a selection of PET biomarker for an early assessment of treatment response.

**Specific Embodiments, Citation of References**

The present invention is not to be limited in scope by specific embodiments described herein. Indeed, various modifications of the invention in addition to those described herein will become apparent to these skilled in the art from the foregoing description and accompanying figures. Such modifications are intended to fall within the scope of the appended claims.

**Claims**

1. A method for screening of positron emission tomography (PET) tracers in early cancer therapy monitoring, comprising:

   (a) providing cultures of multicellular tumor spheroids (MTS),
   (b) treating said MTS cultures with anticancer agent(s) at a predetermined concentration for a predetermined treatment time,
   (c) determining the viable volumes of the MTSs,
   (d) incubating the MTSs with PET tracers of interest for a predetermined period of time,
   (e) measuring total PET tracer uptake for each treatment of the MTSs, and
   (f) analyzing data from PET tracer uptake per unit viable volume of the MTSs and determining suitable PET tracer(s) for each anticancer agent.

2. A method of claim 1, wherein the cancer therapy is breast cancer therapy.

3. A method of claim 1, wherein the viable volumes of the MTSs are determined by semi-automated size determination method (SASDM), comprising:

   (a) imaging a reference object,
   (b) imaging MTS, and
   (c) calculating viable volume of MTS based on imaged total area of MTS, imaged area of necrosis of MTS, imaged total area of the reference object, and known total area of reference object.

4. A method of claim 1, the method further comprises a step of using design of experiment (DOE) methodology to design experiment before step (a) and wherein DOE methodology is used to analyze data in step (f).

5. A method of claim 4, wherein the DOE design comprises factors and response.


**Patentansprüche**

1. Verfahren zum Screening von Positronenemissionstomographie-(PET)-Tracern bei der Frühüberwachung von Krebstherapien, umfassend:

   (a) Bereitstellen von Kulturen multizellulärer Tumorsphäroide (MTS),
   (b) Behandeln der MTS-Kulturen mit Antikrebsmittel(n) mit einer vorbestimmten Konzentration für eine vorbestimmte Behandlungszeit,
   (c) Bestimmen der lebensfähigen Volumen der MTSs,
   (d) Inkubieren der MTSs mit maßgeblichen PET-Tracern für einen vorbestimmten Zeitraum,
   (e) Messen der PET-Tracer-Gesamtaufnahme für jede Behandlung der MTSs, und
   (f) Analysieren von Daten anhand der PET-Tracer-Aufnahme pro Einheit lebensfähigen Volumens der MTSs, und Bestimmen eines geeigneten bzw. geeigneter PET-Tracer für jedes Antikrebsmittel.

2. Verfahren nach Anspruch 1, wobei die Krebstherapie eine Brustkrebstherapie ist.

3. Verfahren nach Anspruch 1, wobei die lebensfähigen Volumen der MTSs bestimmt werden durch ein halbautomatisiertes Größenbestimmungsverfahren (SASDM), umfassend:

   (a) Abbilden eines Bezugsobjekts,
   (b) Abbilden von MTS, und
   (c) Berechnen des lebensfähigen MTS-Volumens auf Grundlage des abgebildeten MTS-Gesamtbereichs, des abgebildeten MTS-Nekrosebereichs, des abgebildeten Gesamtbereichs des Bezugsobjekts und des bekannten Gesamtbereichs des Bezugsobjekts.

4. Verfahren nach Anspruch 1, wobei das Verfahren weiterhin einen Schritt des Einsetzens statistischer Versuchsplanungs-(DOE)-Methodik umfasst, um einen Versuch vor Schritt (a) zu planen, und wobei DOE-Methodik eingesetzt wird, um Daten in Schritt (f) zu analysieren.

**5.** Verfahren nach Anspruch 4, wobei die statistische Versuchsplanung (DOE) Faktoren und Zielgröße umfasst.

**Revendications**

**1.** Procédé de criblage de traceurs de tomographie par émission de positron (TEP) afin d'assurer le contrôle précoce des traitements anticancéreux, comprenant :

(a) préparation de cultures de sphéroïdes de tumeur multicellulaires (MTS),
(b) traitement desdites cultures de sphéroïdes MTS avec un ou des agents anticancéreux à une concentration prédéterminée pendant une durée de traitement prédéterminée,
(c) détermination des volumes viables des sphéroïdes MTS,
(d) incubation des sphéroïdes MTS avec les traceurs de TEP considérés pendant une période prédéterminée,
(e) mesure de l'assimilation totale de traceur de TEP pour chaque traitement des sphéroïdes MTS, et
(f) analyse des données de l'assimilation de traceur de TEP par volume viable unitaire des sphéroïdes MTS, et détermination du ou des traceurs de TEP appropriés pour chaque agent anticancéreux.

**2.** Procédé selon la revendication 1, dans lequel le traitement anticancéreux est un traitement anticancéreux du sein.

**3.** Procédé selon la revendication 1, dans lequel les volumes viables des sphéroïdes MTS sont déterminés par un procédé de détermination de taille semi-automatique (SASDM), comprenant

(a) l'imagerie d'un objet de référence,
(b) l'imagerie des sphéroïdes MTS, et
(c) le calcul du volume viable des sphéroïdes MTS sur la base de la surface totale traitée par imagerie de sphéroïdes MTS, la surface traitée par imagerie de nécrose de sphéroïdes MTS, la surface totale traitée par imagerie de l'objet de référence, et la surface totale connue de l'objet de référence.

**4.** Procédé selon la revendication 1, le procédé comprend, en outre, une étape d'utilisation d'une méthodologie de conception d'expérimentation (DOE) afin de définir l'expérimentation avant l'étape (a) et dans lequel la méthodologie DOE est utilisée afin d'analyser les données à l'étape (f).

**5.** Procédé selon la revendication 4, dans lequel la conception DOE comprend des facteurs et une réponse.

Investigation: PAC (MLR)

Scaled & Centered Coefficients for RA/VOL (Extended)

N=235        R2=0.851        R2 Adj.=0.847
DF=228       Q2=0.841        RSD=11.1348    Conf. lev.=0.95

## FIG.1A

Investigation: PAC (MLR)

RA/VOL predicted for Tracer (adjusted to reference)

N=235        R2=0.851        R2 Adj.=0.847
DF=228       Q2=0.841        RSD=11.1348    Conf. lev.=0.95

## FIG.1B

Investigation: DOC (MLR)

Scaled & Centered Coefficients for RA/VOL (Extended)

N=208    R2=0.879    R2 Adj.=0.876
DF=201    Q2=0.871    RSD=9.2379    Conf. lev.=0.95

## FIG.2A

Investigation: DOC (MLR)

RA/VOL predicted for Tracer (adjusted to reference)

N=208    R2=0.879    R2 Adj.=0.876
DF=201    Q2=0.871    RSD=9.2379    Conf. lev.=0.95

- RA/VOL
- L. Conf. int.
- U. Conf. int.

## FIG.2B

Investigation: DOX2 (MLR)

Scaled & Centered Coefficients for RA/VOL (Extended)

N=145        R2=0.890        R2 Adj.=0.885
DF=138       Q2=0.879        RSD=12.7594   Conf. lev.=0.95

## FIG.3A

Investigation: DOX2 (MLR)

RA/VOL predicted for Tracer (adjusted to reference)

N=145        R2=0.890        R2 Adj.=0.885
DF=138       Q2=0.879        RSD=12.7594   Conf. lev.=0.95

## FIG.3B

Investigation: TAM (MLR)

Scaled & Centered Coefficients for RA/VOL (Extended)

N=205          R2=0.503        R2 Adj.=0.490
DF=199         Q2=0.473        RSD=12.1684    Conf. lev.=0.95

# FIG.4A

Investigation: TAM (MLR)

RA/VOL predicted for Tracer (adjusted to reference)

N=205     R2=0.503    R2 Adj.=0.490
DF=199    Q2=0.473    RSD=12.1684    Conf. lev.=0.95

# FIG.4B

Investigation: Imatinib (MLR)

Scaled & Centered Coefficients for RA/VOL (Extended)

N=211          R2=0.318          R2 Adj.=0.298
DF=204         Q2=0.269          RSD=15.6423   Conf. lev.=0.95

## FIG.5A

Investigation: Imatinib (MLR)

RA/VOL predicted for Tracer (adjusted to reference)

N=211          R2=0.318          R2 Adj.=0.298
DF=204         Q2=0.269          RSD=15.6423   Conf. lev.=0.95

- RA/VOL
- L. Conf. int.
- U. Conf. int.

## FIG.5B

% Viable Volume

FIG.6

% Viable Volume

FIG.7

22

FIG.8

Paclitaxel/MCF-7

**2days treatment**

| Dunnett's Multiple Comparison Test | Mean Diff. | q | P value |
|---|---|---|---|
| Control vs 10nM | −14.89 | 2.516 | P < 0.05 |
| Control vs 100nM | −4.611 | 0.8117 | P > 0.05 |
| Control vs 1M | −2.389 | 0.3934 | P > 0.05 |

**3days treatment**

| Dunnett's Multiple Comparison Test | Mean Diff. | q | P value |
|---|---|---|---|
| Control vs 10nM | 25.90 | 4.460 | P < 0.01 |
| Control vs 100nM | 26.98 | 4.885 | P < 0.01 |
| Control vs 1M | 24.27 | 4.293 | P < 0.01 |

FIG.9A

Paclitaxel/BT474

FIG.9B

Docetaxel/MCF-7

FIG.10A

EP 1 861 713 B1

Docetaxel/BT474

FIG.10B

FIG.11A

Doxorubicin/BT474

2days treatment

3days treatment

| Dunnett's Multiple Comparison Test | Mean Diff. | q | P value |
|---|---|---|---|
| Control vs 10nM | 6.256 | 1.352 | P > 0.05 |
| Control vs 100nM | 15.93 | 3.833 | P < 0.01 |
| Control vs 1M | 15.97 | 3.843 | P < 0.01 |

| Dunnett's Multiple Comparison Test | Mean Diff. | q | P value |
|---|---|---|---|
| Control vs 10nM | 10.72 | 3.043 | P < 0.01 |
| Control vs 100nM | 4.943 | 1.140 | P > 0.05 |
| Control vs 1M | 15.03 | 3.989 | P < 0.01 |

FIG.11B

Tamoxifen/MCF-7

**2days treatment**

FDG-uptake/volume (%of control)

| Dunnett's Multiple Comparison Test | Mean Diff. | q | P value |
|---|---|---|---|
| Control vs 10nM | 1.155 | 0.2136 | P > 0.05 |
| Control vs 100nM | −11.11 | 2.054 | P > 0.05 |
| Control vs 1M | 20.55 | 3.712 | P < 0.01 |

**3days treatment**

FDG-uptake/volume (%of control)

| Dunnett's Multiple Comparison Test | Mean Diff. | q | P value |
|---|---|---|---|
| Control vs 10nM | 0.4933 | 0.1095 | P > 0.05 |
| Control vs 100nM | 6.935 | 1.540 | P > 0.05 |
| Control vs 1M | 16.86 | 3.670 | P < 0.01 |

FIG.12A

EP 1 861 713 B1

**Tamoxifen/BT474**

FIG.12B

EP 1 861 713 B1

Imatinib/MCF-7

FIG.13A

EP 1 861 713 B1

Imatinib/BT474

### 2days treatment

FDG-uptake/volume (%of control)

| Dunnett's Multiple Comparison Test | Mean Diff. | q | P value |
|---|---|---|---|
| Control vs 10nM | −2.127 | 0.4906 | P > 0.05 |
| Control vs 100nM | −2.070 | 0.4607 | P > 0.05 |
| Control vs 1M | −7.042 | 1.676 | P > 0.05 |

### 3days treatment

FDG-uptake/volume (%of control)

| Dunnett's Multiple Comparison Test | Mean Diff. | q | P value |
|---|---|---|---|
| Control vs 10nM | 0.05834 | 0.01585 | P > 0.05 |
| Control vs 100nM | −8.290 | 2.106 | P > 0.05 |
| Control vs 1M | 0.7417 | 0.2015 | P > 0.05 |

## FIG.13B

EP 1 861 713 B1

FIG.14

2697

FIG.15

FIG.16

MCF-7 growth curve

$$y = 0.5889e^{0.0537x}$$
$$R^2 = 0.9545$$

FIG.17

EP 1 861 713 B1

FIG.18

BON growth curve

$y = 0.1501e^{0.1386x}$
$R^2 = 0.9952$

FIG.19

FIG.20

Glioma growth curve

$y = 0.469e^{0.0165x}$
$R^2 = 0.6186$

FIG.21

EP 1 861 713 B1

FIG.22

FIG.23

**REFERENCES CITED IN THE DESCRIPTION**

**Non-patent literature cited in the description**

- **Cohade et al.** *Cancer Journal,* 2002, vol. 8 (2), 119-134
- **van der Hiel et al.** *Journal of Cancer Research and Clinical Oncology,* 2001, vol. 127 (5), 269-277
- **Gambhir.** *Journal of Clinical Oncology,* 2005, vol. 23 (8), 1664-1673
- **Kunz-Schughart et al.** *Cell Biology International,* 1999, vol. 23 (3), 157-161
- **Gati et al.** *J. Nucl. Med.,* 1991, vol. 32 (12), 2258-2265
- **Gati et al.** *Eur. J. Cancer,* 1990, vol. 26 (7), 802-7
- **Aboage et al.** *Anti-Cancer Drug Design,* 1998, vol. 13, 703-730